# EUROPEAN PATENT SPECIFICATION

(11) **EP 2 645 964 B1**
(45) Date of publication and mention of the grant of the patent: **17.06.2015**
(21) Application number: 11807753.6
(22) Date of filing: 30.11.2011
(51) Int. Cl.: A61F 2/30, A61L 27/00

(54) **MULTIFUNCTIONAL PROSTHESIS WITH MULTILAYER COVERING AND METHOD OF PRODUCING THEREOF**
MULTIFUNKTIONSPROTHESE MIT MEHRSCHICHTIGEM ÜBERZUG UND HERSTELLUNGSVERFAHREN DAFÜR
PROTHÈSE MULTIFONCTIONNELLE AVEC REVÊTEMENT MULTICOUCHE ET PROCÉDÉ DE PRODUCTION ASSOCIÉ

(30) Priority: 30.11.2010 IT PD20100360
(43) Date of publication of application: 09.10.2013
(60) Divisional application: 15166772.2
(73) Proprietor: Guerra, Paolo, 20145 Milano (IT); Giuliani, Alessio, 20136 Milano (IT)
(72) Inventor: Guerra, Paolo, 20145 Milano (IT); Giuliani, Alessio, 20136 Milano (IT)
(74) Representative: Postiglione, Ferruccio
(86) International application number: PCT/IB2011/055382
(87) International publication number: WO 2012/073201

(56) References cited:
- WO-A2-2007/076383
- US-A1- 2006 004 466
- US-A1- 2008 255 677

## Description

The present invention relates to a prosthesis, in particular a multifunctional prosthesis with multilayer covering, having an improved and differentiated biological response, and method of producing thereof.

In particular it is known of in the prosthetics sector to implant prostheses, e.g. orthopaedic, trying to favour the osseointegration or the osseous encapsulation of the prosthesis as far as possible.

Osseointegration is essential for restoring the biomechanical functions of the prosthesized bone as soon as possible.

Not infrequently the need arises to service the implanted prosthesis: in practice this involves explanting the prosthesis to replace, reposition or modify it and adapt it better to the specific anatomical conformation of the patient.

The explant phase of the prosthesis is highly invasive in that the prosthesis, thanks to its osseointegration is firmly bound both to the cortex and to the inner spongy part of the bone.

As a result, the removal of prosthesis performed according to the prior art often proves excessively invasive for the patient.

It follows that in the art servicing operations are reduced to a minimum except where strictly necessary.

There is therefore a tendency to leave the prosthesis first implanted in the patient in position which does not always ensure optimal functioning for the patient.

In addition, one of the main causes of failure of the prosthetic implant is due to infections.

Such infections may occur subsequent to the implant and require removal of the prosthesis.

The need is therefore also felt to provide prostheses which limit the infections that are one of the main causes of failure of prosthesis implants.

The purpose of the present invention is to make a prosthesis which overcomes the drawbacks mentioned with reference to the prior art. The closest prior art is disclosed in US 2008/255677.

Such drawbacks and limitations are resolved by a prosthesis according to claim 1 and by a method of making a prosthesis according to claim 16.

Other embodiments of the prosthesis according to the invention are described in the subsequent claims.

Further characteristics and advantages of the present invention will be clearly comprehensible from the description given below of its preferred embodiments, made by way of a non-limiting example, wherein:

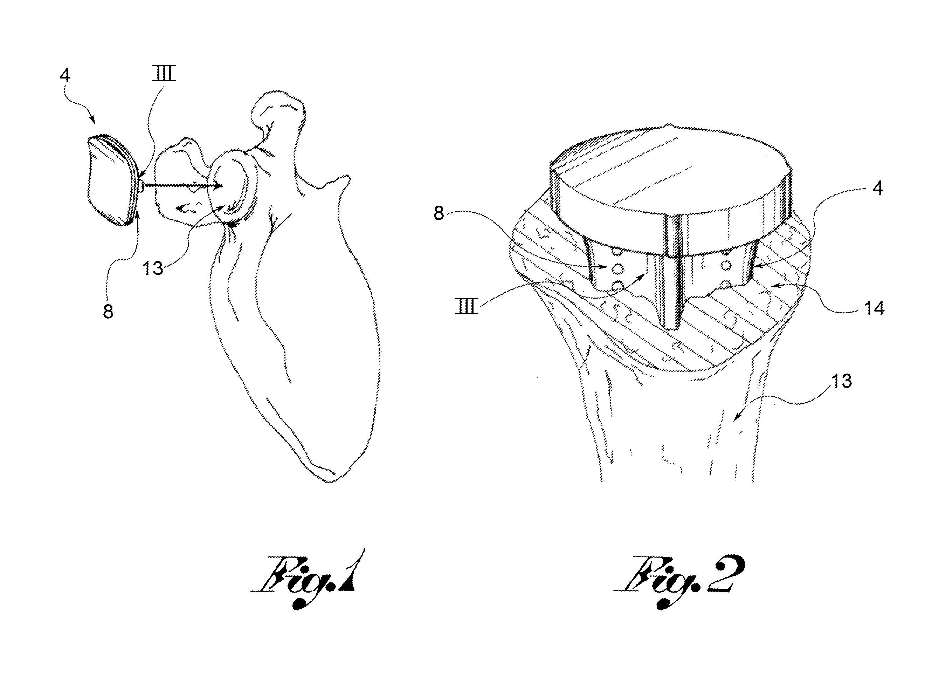

figures 1 and 2 shows perspective views of applications of prostheses according to possible embodiments of the present invention;
figure 3 shows an enlarged schematic view of the details III of the prosthesis in figures 1 and 2, according to one possible embodiment of the present invention;
figures 4-6 shows enlarged schematic views of the detail III in figures 1 and 2, according to embodiment variations of the present invention;
figure 7 shows an enlarged view of the detail VII in figure 6.

The elements or parts of elements common to the embodiments described below will be indicated using the same reference numerals.

With reference to the aforesaid figures, reference numeral 4 globally denotes a multifunctional prosthesis with multilayer covering.

The definition of prosthesis should be considered in the broad, not limited, sense; in other words any prosthesis suitable for interfacing with or replacing at least partially a bone or portion of bone is meant.

Merely by way of example, one might mean a glenoid, shoulder, knee or hip prosthesis, a prosthesis of a vertebral body and the like.

The prosthesis 4 comprises a prosthesis body 8 having at least one interface wall 12 suitable for interfacing with at least one bone tissue,

The term "interface" is taken to mean that the interface wall 12 of the prosthesis body 8, or a portion of it, is suitable for positioning itself in contact with a bone, e.g. with a cortex 13 of the bone, that is the external, rigid portion covering the bone and/or with the spongy part of the bone 14, that is the inner bone tissue covered by said cortex 13.

The interface wall 12 comprises a substrate 16.

According to one embodiment said substrate 16 is made from titanium or titanium alloy, such as a Ti6Al4V alloy, known for its biocompatibility.

The interface wall 12 need not necessarily be solid and in single material but may comprise a core in different material subsequently covered, e.g. titanium or titanium alloy at such substrate 16.

On the side of an associable first covering 20 and on the side opposite the interface wall 12, the substrate 16 comprises protuberances 21 and/or recesses 22.

Such protuberances 21 and/or recesses 22 may, in one embodiment, have a circular geometry e.g. cylindrical or spherical, suitable for encouraging attachment with first functional groups 28, as described further below.

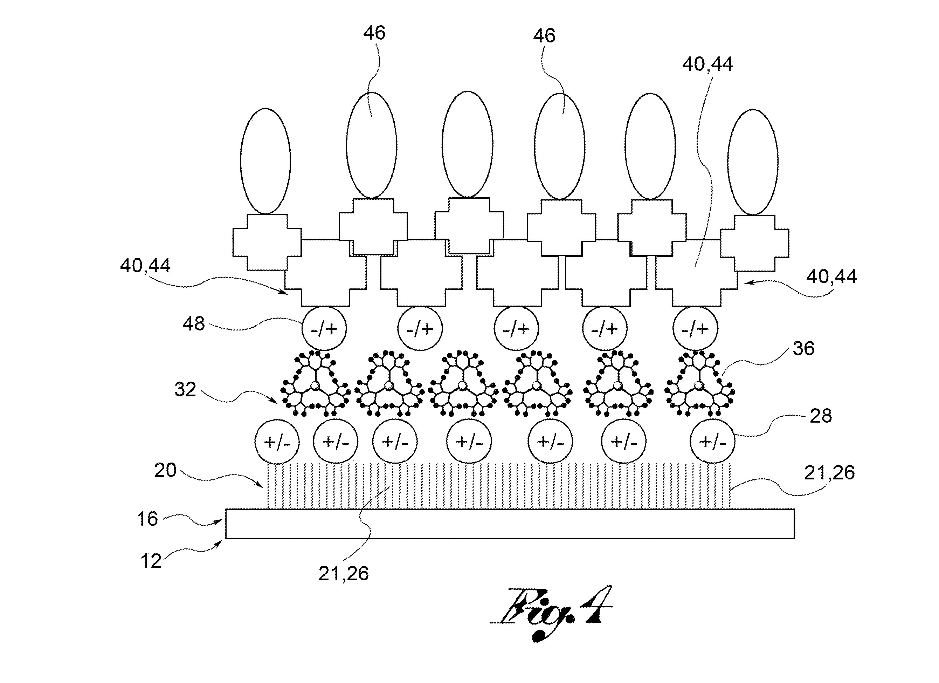

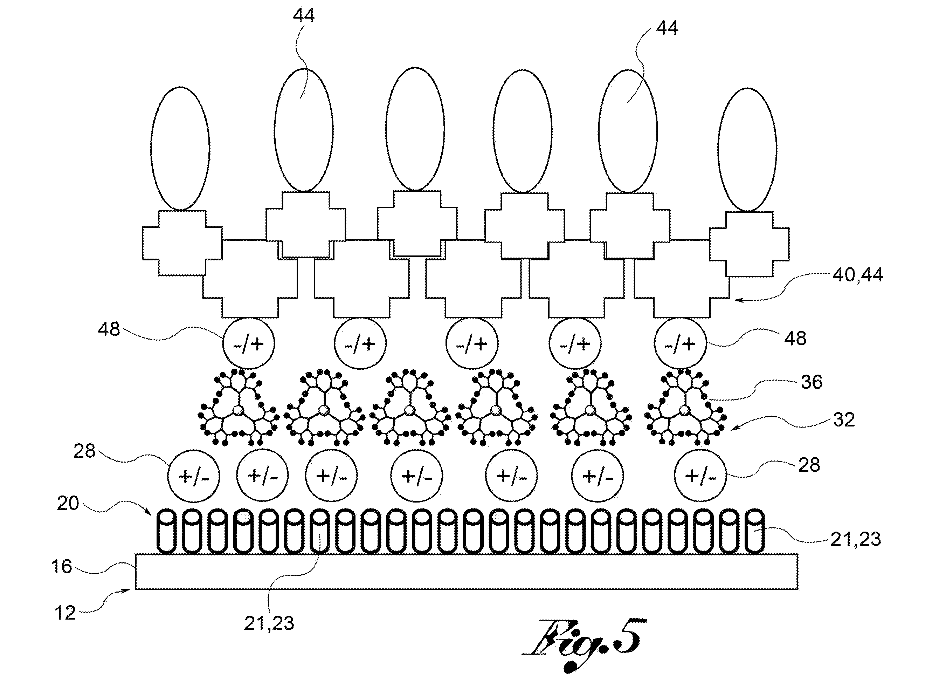

According to one embodiment said protuberances comprise nanotubes 23 or nanowires 26. Said nanotubes 23 or nanowires 26 are mono-dimensional structures, known in the art, schematically illustrated for example in figures 4-5.

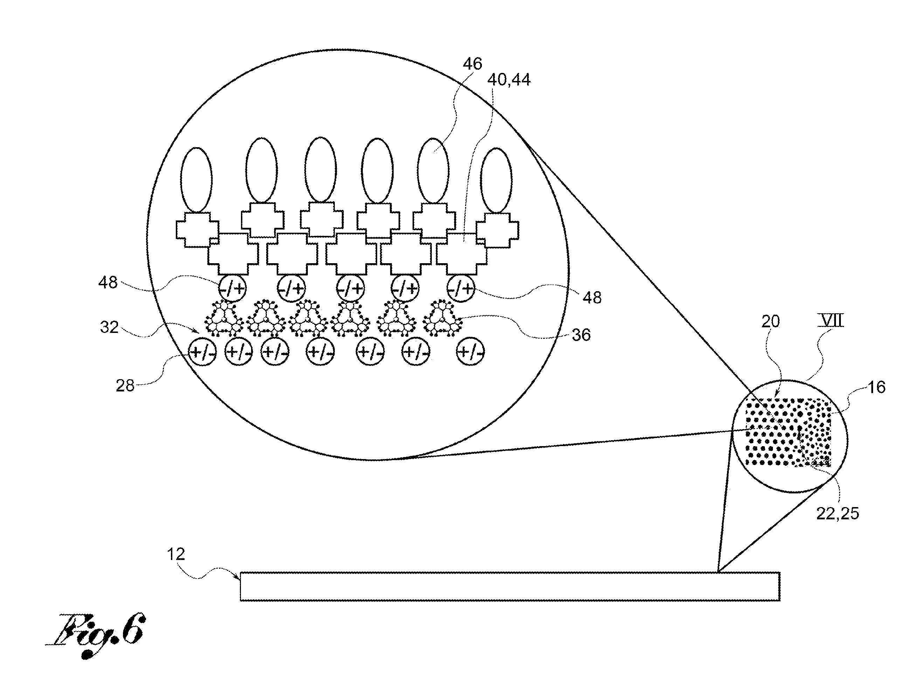

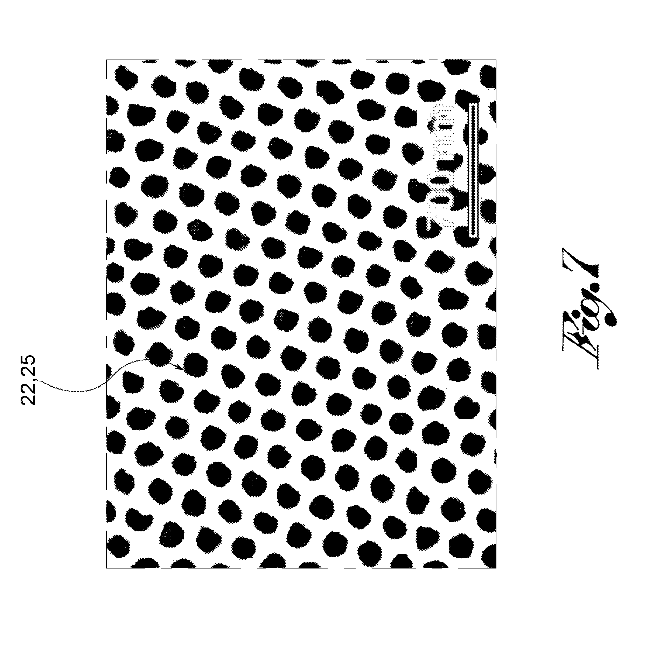

According to one embodiment said recesses 22 comprise nanoholes 25 (figures 6, 7). For example said nanoholes 25 may have a circular geometry.

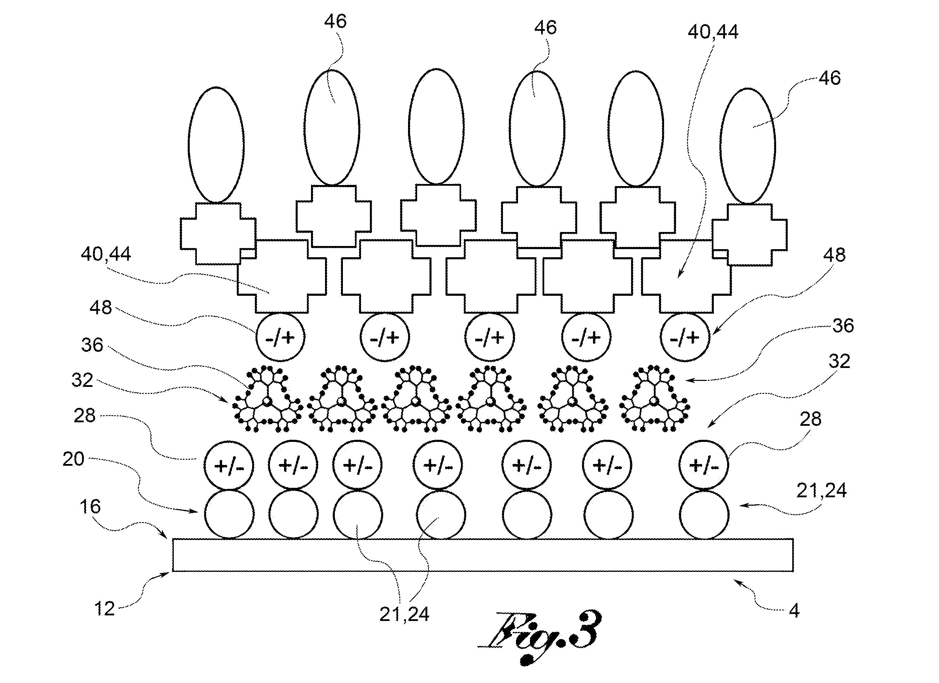

According to a further possible embodiment, on the side of the first covering 20, the protuberances 21 of the substrate 16 comprise nanometric spheres 24 (figure 3).

In particular the spheres have a high surface area: volume ratio, that is to say they have a large number of atoms on the surface which can create a plurality of chemical bonds.

According to one embodiment, such chemical bonds are of the ionic or covalent type.

Preferably, said nanometric spheres 24 are made from TiO2.

Preferably, the nanometric spheres 24 have a diameter of 100nm to 500nm.

A first covering 20 comprising first functional groups 28 suitable to form chemical bonds, is applied to said substrate 16.

Said nanometric spheres 24 are suitable for strengthening the bonds between the substrate 16 and the first functional groups 28.

According to one embodiment, such chemical bonds are of the ionic or covalent type.

According to one embodiment said substrate 16 is activated by the reaction of acids such as H3PO4, H2SO4 suitable for reacting with the material of the substrate so as to obtain the first functional groups 28 of the first covering 20 on it.

Preferably, the first functional groups 28 of the first covering 20 comprise OH-, COOH-, SH-, NH2- groups and the like.

On the same substrate 16 both protuberances 21,23,24,26 and recesses 22, 25 may be provided so as to improve the strength and quantity of bonds with said functional groups.

According to one embodiment a second covering 32 comprising nano-structured ramified polymers 36 is applied to the first covering 20, on the side opposite the substrate 16.

According to one embodiment, the second covering 32 comprises "dendrimer".

The ramified polymers 36 have the function of creating a plurality of chemical bonds with the first functional groups 28 of the first covering 20 as well as the function of creating bonds with further layers to superpose on the second covering 32.

Advantageously, an interface covering 40 comprising molecules 44 suitable for encouraging and /or discouraging the osseointegration of the prosthesis 4 depending on the greater or less osseointegration required of the interface wall 12 or portions thereof is applied to said second covering 32 on the side opposite the first covering 20.

In other words, the molecules 44 of the interface covering 40 are "calibrated" so as to provide specific biological stimuli when interfaced with bone cells 46; consequently depending on the type of molecules 44 selected and applied, the bone cells may be attracted or repelled.

Preferably, second functional groups 48 suitable to form chemical bonds are inserted between the interface covering 40 and the second covering 32.

According to one embodiment, such chemical bonds are of the ionic or covalent type.

For example, the second functional groups 48 comprise OH-, COOH-, SH-, NH2- groups and the like.

Said second functional groups 48 have the function of strengthening the bonds between the second covering 32 and the interface covering 40.

According to one embodiment the molecules 44 of the interface covering 40 comprise amino acids of the type RGD (Arg-Gly-Asp) and/or fibronectin suitable for favouring the osseointegration of the prosthesis 4.

According to a further embodiment, the molecules 44 of the interface covering 40 comprise monoclonal antibodies (TNF inhibitors) suitable for discouraging osseointegration.

The molecules 44 of the interface covering 40 may also comprise PEG and/or chitosan suitable for discouraging the risk of infections.

A method of making a prosthesis according to the present invention will now be described.

In particular the interface wall 12 having a substrate 16 is predisposed and the nanometric spheres are applied to the substrate 16.

For example, the substrate 16 is in titanium or titanium alloy and the nanometric spheres are in TiO2.

Such application of nanometric spheres is preferably performed by means of a PVD technique such as sputtering.

Then, according to one possible embodiment, the surface of the substrate is activated. For example, the surface of the substrate 16 is activated by applying acids, such as H3PO4, H2SO4. The first covering 20 comprising the first functional groups 28 suitable to form chemical bonds is obtained following such activation of the substrate 16. Said chemical bonds are for example of the ionic or covalent type.

The second covering 32 comprising nanostructures is then applied.

For example, said nanostructures comprise nano-structured ramified polymers 36. The interface covering 40 comprising molecules 44 is then applied to the second covering 2.

Second functional groups 48 suitable to form chemical bonds are preferably inserted between the interface covering 40 and the second covering 32.

Said chemical bonds are for example of the ionic or covalent type.

According to one embodiment different molecules 44 are applied to separate portions of the interface covering 40 depending on the desired level of osseointegration.

It is for example possible to encourage osseointegration in some portions of the prosthesis body 8 which need to integrate in the shortest time possible, and at the same time discourage such integration in different portions of the prosthesis body 8, for example so as to facilitate, or render less invasive, any subsequent removal or explant of the prosthesis.

Lastly, according to one possible embodiment, the interface covering 40 comprises PEG and/or chitosan molecules suitable to discourage the risk of infection following implant.

As may be appreciated from the description, the prosthesis according to the invention makes it possible to overcome the drawbacks presented in the prior art.

In particular the prosthesis according to the invention is of the multifunctional type, that is can be calibrated to behave differently towards the cells with which it comes into contact.

In fact, the interface covering may be modified by applying molecules able to encourage or discourage the osseointegration of the prosthesis or portions thereof. In fact, the type of molecules applied may attract or not the osteoblasts and cells which, after the implant, come into contact with the interface covering.

In other words, the molecules present on the interface covering are able to locally influence the behaviour of the prosthesis in relation to the cells with which it interfaces so as to accelerate osseointegration with specific portions of bone and/or discourage osseointegration with tissues with which it is preferable for the prosthesis not to bond, for example so as to facilitate any explants and substitutions of the prosthesis.

The particular succession of coverings on the substrate is able to contemporarily guarantee the multifunctionality of the prosthesis and also the solidness of the bonds and their resistance over time.

In other words, the molecules of the various coverings are bound to each other by numerous strong chemical bonds.

Moreover, by applying suitable molecules to the interface covering, it is possible to avoid side effects, such as infections or rejection phenomena of the prosthesis.

A person skilled in the art may make numerous modifications and variations to the prosthesis described above so as to satisfy contingent and specific requirements, all contained within the sphere of protection of the invention as defined by the appended claims.

## Claims

1. Multifunctional prosthesis (4) with multilayer covering comprising
- a prosthesis body (8) having at least one interface wall (12) suitable for interfacing with at least one bone tissue,
- the interface wall (12) comprising a substrate (16) **characterised in that**
- a first covering (20) comprising first functional groups (28) suitable to form chemical bonds, is applied to said substrate (16),
- wherein, on the side of the first covering (20) the substrate (16) comprises protuberances (21,23,24,26) and/or recesses (22,25) suitable to favour the attachment of said first functional groups (28),
- a second covering (32) comprising nanostructures being applied to said first covering (20), on the side opposite the substrate (16),
- an interface covering (40) comprising molecules (44) being applied to said second covering (32) on the side opposite the first covering (20),
- wherein second functional groups (48) suitable to form chemical bonds, so as to strengthen the bonds between the second covering (32) and the interface covering (40) are inserted between the interface covering (40)and the second covering (32).

2. Prosthesis (4) according to claim 1, wherein said substrate (16) is made from titanium or titanium alloy.

3. Prosthesis (4) according to claim 1 or 2 wherein said protuberances (21) comprise nanotubes (23) and/or nanowires (26).

4. Prosthesis (4) according to claim 1,2 or 3 wherein on the side of the first covering (20), said protuberances (21) comprise nanometric spheres (24) suitable to strengthen the bonds between the substrate (16) and the first functional groups (28).

5. Prosthesis (4) according to claim 4, wherein said nanometric spheres (24) have a diameter of 100nm to 500nm.

6. Prosthesis (4) according to claim 4 or 5 wherein said nanometric spheres are made from TiO2.

7. Prosthesis (4) according to any of the previous claims, wherein said recesses (22) comprise nanoholes (25).

8. Prosthesis (4) according to any of the previous claims, wherein the first functional groups (48) of the first covering comprise OH-, COOH-, SH-, NH2- groups.

9. Prosthesis (4) according to any of the previous claims, wherein the second covering (32) comprises nano-structured ramified polymers (36).

10. Prosthesis (4) according to any of the previous claims, wherein said second covering (32) comprises "dendrimer".

11. Prosthesis (4) according to any of the previous claims, wherein the second functional groups (48) comprise OH-, COOH-, SH-, NH2- groups.

12. Prosthesis (4) according to any of the previous claims, wherein the molecules (44) of the interface covering (40) comprise amino acids of the type RGD (Arg-Gly-Asp) and/or fibronectin suitable to favour the osseointegration of the prosthesis (4).

13. Prosthesis (4) according to any of the previous claims, wherein the molecules (44) of the interface covering (40) comprise monoclonal antibodies (TNF inhibitors) suitable to discourage osseointegration.

14. Prosthesis (4) according to any of the previous claims, wherein the molecules (44) of the interface covering (40) comprise PEG and/or chitosan suitable to discourage the risk of infections.

15. Method of producing a prosthesis (4) according to any of the previous claims, comprising the steps of:
- preparing an interface wall (12) having a substrate (16)
- applying protuberances (21,23,24,26) and/or recesses (22,25) to the substrate (16), suitable to favour the attachment of the first functional groups (28),
- activating the surface of the substrate (16) by applying chemical substances, so as to obtain the first covering (20) comprising first functional groups (28) suitable to form chemical bonds,
- applying the second covering (32) comprising nanostructures, inserting second functional groups (48) able to form chemical bonds between the interface covering (40)and the second covering (32), applying the interface covering (40) comprising molecules (44) to the second covering (32).

## Patentansprüche

1. Multifunktionsprothese (4) mit einem mehrschichtigen Überzug, die umfasst:
- einen Prothesenkörper (8) mit zumindest einer Verbindungswand (12), die dazu geeignet ist, sich mit zumindest einem Knochengewebe zu verbinden,
- die Verbindungswand (12), die ein Substrat (16) umfasst,
**dadurch gekennzeichnet, dass**
- ein erster Überzug (20), der erste funktionelle Gruppen (28) umfasst, die dazu geeignet sind, chemische Bindungen zu bilden, auf das Substrat (16) aufgetragen ist,
- wobei das Substrat (16) auf der Seite des ersten Überzugs (20) Vorsprünge (21, 23, 24, 26) und/oder Ausnehmungen (22, 25) umfasst, die sich dazu eignen, die Anhaftung der ersten funktionellen Gruppen (28) zu begünstigen,
- ein zweiter Überzug (32), der Nanostrukturen umfasst, auf den ersten Überzug (20) auf der dem Substrat (16) gegenüberliegenden Seite aufgetragen ist,
- ein Verbindungsüberzug (40), der Moleküle (44) umfasst, auf den zweiten Überzug (32) auf der dem ersten Überzug (20) gegenüberliegenden Seite aufgetragen ist,
- wobei zweite funktionelle Gruppen (48), die dazu geeignet sind, chemische Bindungen zu bilden, um die Bindungen zwischen dem zweiten Überzug (32) und dem Verbindungsüberzug (40) zu festigen, zwischen dem Verbindungsüberzug (40) und dem zweiten Überzug (32) eingefügt sind.

2. Prothese (4) nach Anspruch 1, wobei das Substrat (16) aus Titan oder Titanlegierung hergestellt ist.

3. Prothese (4) nach Anspruch 1 oder 2, wobei die Vorsprünge (21) Nanoröhrchen (23) und/oder Nanodrähte (26) umfassen.

4. Prothese (4) nach Anspruch 1, 2 oder 3, wobei die Vorsprünge (21) auf der Seite des ersten Überzugs (20) nanometrische Kugeln (24) umfassen, die dazu geeignet sind, die Bindungen zwischen dem Substrat (16) und den ersten funktionellen Gruppen (28) zu festigen.

5. Prothese (4) nach Anspruch 4, wobei die nanometrischen Kugeln (24) einen Durchmesser von 100 nm bis 500 nm aufweisen.

6. Prothese (4) nach Anspruch 4 oder 5, wobei die nanometrischen Kugeln aus TiO2 hergestellt sind.

7. Prothese (4) nach einem der vorstehenden Ansprüche, wobei die Ausnehmungen (22) Nanolöcher (25) umfassen.

8. Prothese (4) nach einem der vorstehenden Ansprüche, wobei die ersten funktionellen Gruppen (48) des ersten Überzugs OH-, COOH-, SH- und NH2-Gruppen umfassen.

9. Prothese (4) nach einem der vorstehenden Ansprüche, wobei der zweite Überzug (32) nanostrukturierte verzweigte Polymere (36) umfasst.

10. Prothese (4) nach einem der vorstehenden Ansprüche, wobei der zweite Überzug (32) "Dendrimer" umfasst.

11. Prothese (4) nach einem der vorstehenden Ansprüche, wobei die zweiten funktionellen Gruppen (48) OH-, COOH-, SH- und NH2-Gruppen umfassen.

12. Prothese (4) nach einem der vorstehenden Ansprüche, wobei die Moleküle (44) des Verbindungsüberzugs (40) Aminosäuren vom Typ RGD (Arg-Gly-Asp) und/oder Fibronektin umfassen, dazu geeignet, die Knochenverankerung der Prothese (4) zu begünstigen.

13. Prothese (4) nach einem der vorstehenden Ansprüche, wobei die Moleküle (44) des Verbindungsüberzugs (40) monoklonale Antikörper (TNF-Inhibitoren) umfassen, die dazu geeignet sind, eine Knochenverankerung zu verhindern.

14. Prothese (4) nach einem der vorstehenden Ansprüche, wobei die Moleküle (44) des Verbindungsüberzugs (40) PEG und/oder Chitosan umfassen, das dazu geeignet ist, ein Infektionsrisiko zu verhindern.

15. Verfahren zum Herstellen einer Prothese (4) nach einem der vorstehenden Ansprüche, das die Schritte umfasst:
- Herstellen einer Verbindungswand (12) mit einem Substrat (16),
- Vorsehen von Vorsprüngen (21, 23, 24, 26) und/oder Ausnehmungen (22, 25) auf dem Substrat (16), die dazu geeignet sind, die Anhaftung der ersten funktionellen Gruppen (28) zu begünstigen,
- Aktivieren der Oberfläche des Substrats (16) durch Anwenden von chemischen Substanzen, um den ersten Überzug (20) zu erhalten, der erste funktionelle Gruppen (28) umfasst, die dazu geeignet sind, chemische Bindungen zu bilden,
- Aufbringen des zweiten Überzugs (32), der Nanostrukturen umfasst, Einfügen von zweiten funktionellen Gruppen (48), die in der Lage sind, chemische Bindungen zwischen dem Verbindungsüberzug (40) und dem zweiten Überzug (32) zu bilden, Aufbringen des Verbindungsüberzugs (40), der die Moleküle (44) umfasst, auf den zweiten Überzug (32).

## Revendications

1. Prothèse multifonctionnelle (4) avec multicouche comprenant
- un corps de prothèse (8) avec au moins une paroi d'interface (12) adaptée à servir d'interface avec au moins un tissu osseux,
- la paroi d'interface (12) comprenant un substrat (16) **caractérisée en ce que**
- une première couverture (20) comprenant de premiers groupes fonctionnels (28) adaptés à former des liaisons chimiques est appliquée sur ledit substrat (16),
- où, sur le côté de la première couverture (20), le substrat (16) comprend des protubérances (21,23,24,26) et/ou des creux (22,25) adaptés à favoriser l'attachement desdits premiers groupes fonctionnels (28),
- une deuxième couverture (32) comprenant des nanostructures appliquées sur ladite première couverture (20), sur le côté opposé au substrat (16),
- une couverture d'interface (40) comprenant des molécules (44) appliquées à ladite deuxième couverture (32) sur le côté opposé à la première couverture (20),
- où les deuxièmes groupes fonctionnels (48) adaptés à former des liaisons chimiques afin de renforcer les liaisons entre la deuxième couverture (32) et la couverture d'interface (40), sont insérés entre la couverture d'interface (40) et la deuxième couverture (32).

2. Prothèse (4) selon la revendication 1, où ledit substrat (16) est fait en titane ou en alliage de titane.

3. Prothèse (4) selon la revendication 1 ou 2, où ladite protubérance (21) comprend des nanotubes (23) et/ou des nanofils (26).

4. Prothèse (4) selon la revendication 1, 2 ou 3 où, du côté de la première couverture (20), lesdites protubérances (21) comprennent des sphères nanométriques (24) adaptées à renforcer les liaisons entre le substrat (16) et les premiers groupes fonctionnels (28).

5. Prothèse (4) selon la revendication 4, dans laquelle lesdites sphères nanométriques (24) ont un diamètre de 100 nm à 500 nm.

6. Prothèse (4) selon la revendication 4 ou 5, dans laquelle lesdites sphères nanométriques sont en TiO₂.

7. Prothèse (4) selon l'une quelconque des revendications précédentes, dans laquelle lesdits creux (22) comprennent des nano-trous (25).

8. Prothèse (4) selon l'une quelconque des revendications précédentes, dans laquelle les premiers groupes fonctionnels (48) de la première couverture comprennent les groupes OH-, COOH-, SH-, NH₂-.

9. Prothèse (4) selon l'une quelconque des revendications précédentes, dans laquelle la deuxième couverture (32) comprend des polymères ramifiés nano-structurés (36).

10. Prothèse (4) selon l'une quelconque des revendications précédentes, dans laquelle ladite deuxième couverture (32) comprend des « dendrimères ».

11. Prothèse (4) selon l'une quelconque des revendications précédentes, dans laquelle les deuxièmes groupes fonctionnels (48) comprennent les groupes OH-, COOH-, SH-, NH₂-.

12. Prothèse (4) selon l'une quelconque des revendications précédentes, dans laquelle les molécules (44) de la couverture d'interface (40) comprennent des acides aminés du type RGD (Arg-Gly-Asp) et/ou de la fibronectine adaptée à favoriser l'ostéo-intégration de la prothèse (4).

13. Prothèse (4) selon l'une quelconque des revendications précédentes, dans laquelle les molécules (44) de la couverture d'interface (40) comprennent des anticorps monoclonaux (inhibiteurs du TNF) adaptés à empêcher l'ostéo-intégration.

14. Prothèse (4) selon l'une quelconque des revendications précédentes, dans laquelle les molécules (44) de la couverture d'interface (40) comprennent du PEG et/ou du chitosane adapté à empêcher le risque d'infections.

15. Méthode de fabrication de prothèse (4) selon l'une quelconque des revendications précédentes, comprenant les étapes de :
- préparation d'une paroi d'interface (12) ayant un substrat (16)
- application de protubérances (21,23,24,26) et/ou de creux (22,25) au substrat (16), adaptés à favoriser l'attachement des premiers groupes fonctionnels (28),
- activation de la surface du substrat (16) par l'application de substances chimiques, de façon à obtenir la première couverture (20) comprenant les premiers groupes fonctionnels (28) adaptés à former des liaisons chimiques,
- application de la deuxième couverture (32) comprenant des nanostructures, insertion des deuxièmes groupes fonctionnels (48) aptes à former des liaisons chimiques entre la couverture d'interface (40) et la deuxième couverture (32), application de la couverture d'interface (40) comprenant des molécules (44) à la deuxième couverture (32).
